Europäisches Patentamt

(19) European Patent Office

Office européen des brevets

(11) Publication number : **0 208 766**
**B1**

(12) **EUROPEAN PATENT SPECIFICATION**

(45) Date of publication of patent specification :
**28.03.90**

(21) Application number : **86900896.1**

(22) Date of filing : **03.01.86**

(86) International application number :
**PCT/US 86/00023**

(87) International publication number :
**WO/86040 (17.07.86 Gazettee 86/17)**

(51) Int. Cl.⁵ : **B 65 B 43/12**

(54) PACKAGING APPARATUS AND METHOD.

(30) Priority : **04.01.85 US 688696**

(43) Date of publication of application :
**21.01.87 Bulletin 87/04**

(45) Publication of the grant of the patent :
**28.03.90 Bulletin 90/13**

(84) Designated contracting states :
**AT BE CH DE FR GB IT LI LU NL SE**

(56) References cited :
GB--A-- 2 039 265
US--A-- 3 754 370
US--A-- 3 938 299
US--A-- 3 948 015
US--A-- 4 241 562
US--A-- 4 284 221
US--A-- 4 336 681
US--A-- 4 493 684

(73) Proprietor : **AUTOMATED PACKAGING SYSTEMS, INC.**
**8400 Darrow Road**
**Twinsburg Ohio 44087 (US)**

(72) Inventor : **LERNER, Bernard**
**2220 Main Street**
**Peninsula, OH 44264 (US)**
Inventor : **LIEBHART, Dana**
**1616 14th Street**
**Cuyahoga Falls, OH 44221 (US)**

(74) Representative : **Fisher, Bernard et al**
**Raworth, Moss & Cook 36 Sydenham Road**
**Croydon Surrey CRO 2EF (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Technical Field

The present invention relates generally to packaging systems and in particular to a method and apparatus for loading and separating a container from a chain or web of containers.

### Background Art

Various methods and apparatus for packaging articles in plastic bags are available today or have been suggested in the past. In one packaging method, the bags form part of a continuous plastic web, each bag being connected to a contiguous bag along a line of weakness. Typically, the bags define an opening on one face through which the bag is loaded.

In early bagging machines, an operator manually loaded the product into the bag and the bag was pulled downwardly to position the next bag at the loading station. The loaded bag was then manually severed from the web.

Machines and methods for automatically loading a chain of interconnected plastic bags have been developed or have been suggested by the prior art. In general, these machines included a mechanism for sequentially feeding a lead bag to a loading station ; a mechanism for expanding the mouth of the bag and maintaining it in the expanded condition during a loading operation ; and, a mechanism for severing the loaded bag from the chain. After the loaded bag is severed, the packaging sequence begins again with the next bag.

The individual bags are usually joined to the chain or web by a line of weakness generally formed by a plurality of perforations. After the bag is loaded, it is severed from the web along the perforations. Various mechanisms for automatically severing the loaded bag from the web have been developed or suggested. In one known method, the separation along the perforations is initiated by a projection that begins the tearing action near the center of the line of weakness. Severance of the bag then begins at the center of the line of weakness and proceeds outwardly toward the marginal edges. example of such a mechanism is shown in U.S. patent No. 3,477,196, which is owned by the present assignee.

An alternate method for severing a loaded bag from the web is disclosed in patent No. 4,202,153 which is also owned by the present assignee. In the method and apparatus shown in this patent, a transversely movable product carrier enters an opened bag, positioned horizontally, and simultaneously loads the bag and severs it from the web. Severance is achieved by overdriving the product carrier so that it engages the bottom of the bag and drives it away from the web while the remainder of the web is held stationary, thus tearing the loaded bag from the web. In the disclosed apparatus, the perforation breakage commences in portions near the marginal edges of the web and advances inwardly from both portions toward the center. Because the perforations are broken serially, the force needed to sever the container is less than that required if the perforations were broken simultaneously.

In patent No. 3,815,318 (also owned by the present assignee), a packaging method and apparatus is disclosed which illustrates another apparatus for severing a loaded bag along a line of weakness. In this apparatus, the tearing action is produced by a pivoting mechanism which engages a loaded bag and pivots the bag about an axis located near one marginal edge while the web is held stationary. The tearing action then commences at a remote marginal portion and advances towards the edge of the bag that is located at or near the pivot axis.

US-A-4 241 562 discloses a method and apparatus for the automatic filling of plastic bags, according to the first parts of Claims 1 and 7. A web of interconnected bags is advanced along a path of travel successively to position each bag at a loading station where the bag is opened by a blast of compressed air. A container loading mechanism cooperates with the expanded open bag to funnel a product into the bag. The loading mechanism includes a filling piston which reciprocates to drive a product into the bag and which cooperates with a funnel-like structure to allow the jaws of the funnel-like structure to move towards one another and be received in the open bag when the piston is retracted and to expand the jaws during the filling stroke of the piston to further open the mouth of the bag. The filling piston continues its stroke in the filling direction to push the filled bag to a transport position after having separated it from the stationary web along a line of perforations. On the return stroke of the filling piston, the web is advanced by one step to bring the next bag into position and the filling piston actuates a valve to cause a nozzle to be supplied with compressed air to open the bag.

The methods and apparatus described in the above referenced patents all perform satisfactorily for most if not all bagging operations. The disclosed methods and machines have been used to package a variety of articles such as literature, industrial fasteners, and other industrial products. Recently, it has become desirable to provide a means for packaging slaughtered poultry in a quick and reliable manner.

Poultry has become a very popular staple throughout the world. The consumption of poultry has increased significantly over the years. The substantial increase in consumption is due in part to the cost effective manner in which poultry is brought to market. The process has grown more automated over the years and further automation is needed.

It is believed that packaging equipment currently available or suggested in the past could not readily package poultry parts without substantial modification or alternately could not operate reliably at the required packaging rates especially when subjected to the rather harsh environment encountered in a poultry processing plant. It must be remembered that apparatus used in this type of application must be easily cleaned, which in most cases is done with a high temperature, high pressure water spray.

The present invention provides a new and improved packaging apparatus and method for sequentially loading and severing bags from a chain or web of interconnected bags. The disclosed apparatus and method is particularly adapted for automatically packaging poultry discharged by a poultry cutting machine. The apparatus is capable of high speed and reliable operation even in the environment of a poultry processing plant.

According to one aspect of the invention there is provided a packaging machine for loading and separating a bag-like container from a chain or web of containers, comprising

a) structure establishing a path of travel for a web of interconnected bag-like containers,

b) means for advancing said web along said path to successively position each of said containers at a loading station,

c) means for expanding a container positioned at said loading station, and

d) a container loading mechanism comprising funnel means cooperable with the expanded container and operative to funnel a product into the container, characterised by

e) said funnel means being movable into said expanded container,

f) gripper means operative to immobilize said container at said loading station to inhibit relative movement between said container and said loading mechanism and

g) container separating means for severing said container from said web, comprising means for reversing movement in said web while said container is immobilized, said separating means being operative to retract the web to cause separation along a line of weakness connecting said loaded container to the web.

According to a second aspect of the invention there is provided a method as specified in Claim 7.

In the illustrated and preferred construction, the container loading mechanism includes a funnel arrangement defined by a pair of pivotally mounted horns. As the container or bag is fed to the loading position, the horns are rotated to a position spaced from the opening of the bag. Once the mouth of the bag is opened, the horns are pivoted towards the bag such that end portions of the horns enter the mouth of the bag. Together the horns define a funnel-like structure when they are in the product loading position.

During the loading operation, the bag being loaded is substantially immobilized by a gripper mechanism.

In the preferred embodiment, the gripper mechanism comprises a gripper assembly associated with each horn. The gripper assemblies are located near the end portions of the horns and each includes an extendable gripper pad which is operative to clamp a portion of the bag against the side of the horn. When the gripper assemblies are energized, each pad is extended into abutting contact with its associated horns. Since the grippers are energized after the horns enter the mouth of the bag, side regions of the bag are clamped between the bag and its associated horn. With this arrangement, loading forces exerted on the bag by the product being loaded are isolated from the rest of the chain or web.

The individual bags are joined to the web by a lines of weakness preferably formed by a plurality of perforations. In the preferred method, the loaded bag is severed from the web by retracting or reverse feeding the web while the loaded bag remains clamped to the horns. In other words, the direction of web feed is reversed while preventing movement in the loaded bag thus causing the web to sever itself from the loaded bag. After loading and severing the bag, the grippers are retracted, allowing the loaded bag to fall from the loading horns and into a receptacle or on to a conveyor, etc. for further processing or shipment.

According to the exemplary embodiment, the bag being loaded is oriented vertically so that the product is loaded into the bag by gravity. By using the disclosed gripper/horn mechanism, the forces exerted by the product as it falls into the bag are isolated from the rest of the web and thus premature separation along the lines of weakness (which define the individual bags) is inhibited.

A conveyor system, including a pinch roller assembly is operative to position a lead bag in the web at the loading station. The pinch roller assembly includes an externally driven conveyor roller and a feed roller biases into engagement with the conveyor roller. The feed roller is frictionally driven by the conveyor roller. A transmission, constructed in accordance with a preferred embodiment of the invention controls the direction of roller rotation and is actuatable to either advance or retract at least a portion of the web. In the preferred embodiment, retraction of the web is achieved by a mechanism that imparts an incremental reverse rotation to the pinch roller assembly to produce a slight retraction in the web.

In the illustrated embodiment, the transmission includes one or more chains or belts for transferring rotation from a drive source such as an electric motor to the pinch roller. To minimize slippage and to enhance durability, in the disclosed embodiment, chains are used as power transfer elements. In the disclosed construction, primary and secondary chain systems are employed. The primary chain connects the drive motor to a clutch assembly which in turn controls the coupling of the primary chain drive to the secondary chain drive. The conveyor roller is driven by the secondary chain drive. With the preferred method, a bag is advanced to the loading station by actuation of the clutch assembly to interconnect the primary and secondary chain drives to produce continuous rotation in the conveyor roller until the lead bag in the web is fed to the loading position.

As indicated above, the loaded bag is severed by retracting the web slightly while the bag remains clamped to the loading horns. In the preferred method, retraction of the web is achieved by reverse rotation of the conveyor roller. Although reversing the direction of roller rotation by reversal of the drive motor is contemplated by the present invention, in the preferred embodiment, the reverse rotation of the roll is achieved by a fluid pressure operated actuator connected to a slidable shuttle block that forms part of the secondary chain drive. In particular, the slidable shuttle carries a pair of sprockets connected to the secondary chain. The segment of the second chain coupled to the sprockets drivingly engages the conveyor roller.

When retraction of the web is desired, the drive motor is declutched from the secondary chain drive. The segment of the chain, connected to the clutch assembly, is immobilized preferably by a brake forming part of the assembly which, when energized, locks an output drive sprocket (of the clutch assembly) to inhibit rotation.

The fluid pressure operated actuator is then actuated to displace the shuttle block causing the chain segment coupled to the shuttle sprockets to translate. The conveyor roller being coupled to the translating segment of the chain is caused to reverse rotate slightly, thus causing retraction of the web and ultimately severance of the loaded bag. With the disclosed arrangement, extremely rapid cycle times are achievable without sacrificing the reliability of the overall feed mechanism.

According to a feature of the invention, the bagging apparatus includes an accumulator for holding products while a bag is being positioned at the loading station. The accumulator is especially useful in a poultry packaging application for it enables two or more poultry carcasses to be accumulated and loaded into a single bag. In the preferred embodiment, the accumulator is defined by a funnel-like receptacle having a pivotally mounted exit door. The exit door, preferably located at the base of the accumulator, is opened and closed by a fluid pressure operated actuator in synchronization with the bag positioning and horn actuating mechanism.

Additional features of the invention will become apparent and a fuller understanding obtained by reading the following detailed description made in connection with the accompanying drawings.

Brief Description of Drawings

Figure 1 is a front elevational view of a bagging apparatus constructed in accordance with a preferred embodiment of the invention ;

Figure 2 is a fragmentary, side elevational view of the bagging apparatus shown in Figure 1 ;

Figure 3 is a side elevational view of an accumulator forming part of the bagging apparatus shown in Figure 1 ;

Figure 4 is another fragmentary, side elevational view of the bagging apparatus ;

Figure 5 is a rear, elevational view of a feed mechanism forming part of the bagging apparatus ;

Figure 6 is side elevational view of a feed roller drive mechanism forming part of the bagging apparatus ;

Figure 7 is a fragmentary view as seen from the plane indicated by the lines 7-7 in Figure 6 ;

Figure 8 is a fragmentary view as seen from the plane indicated by the lines 8-8 in Figure 6 ;

Figure 9 is a fragmentary view as seen from the plane indicated by the lines 9-9 in Figure 6 ;

Figure 10 is a side view of the feed roll drive mechanism shown in Figure 6 ; and,

Figures 11-16 illustrate, schematically, the packaging sequence provided by the disclosed bagging apparatus.

Best Mode for Carrying Out the Invention

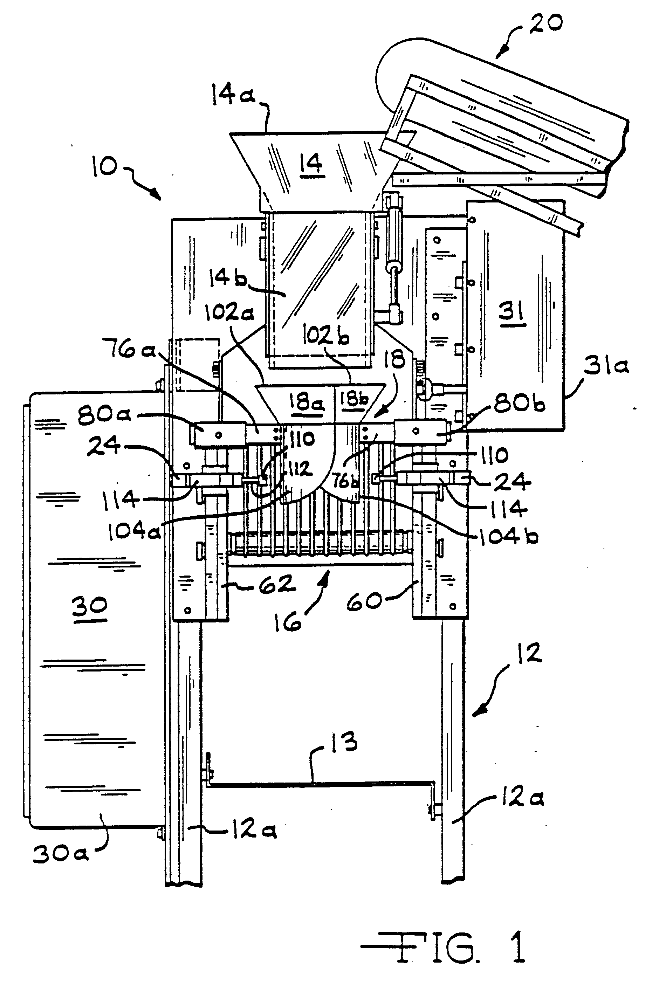

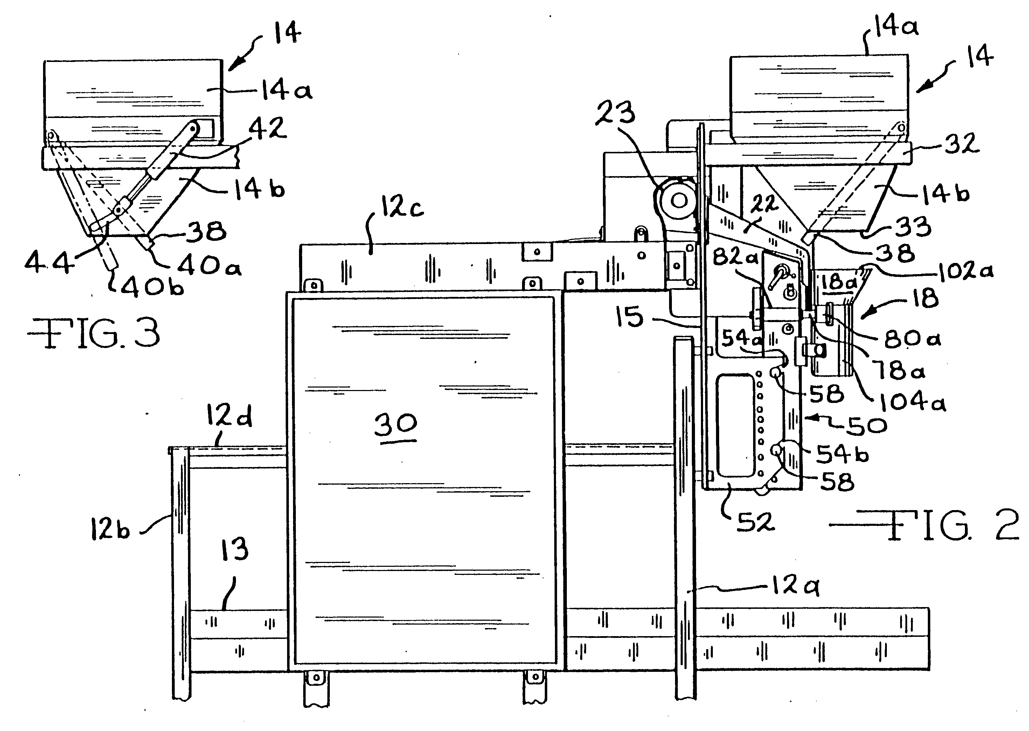

Figure 1 illustrates an overall view of a bagging apparatus 10 constructed in accordance with the preferred embodiment of the invention. Referring also to Figure 2, the bagger 10 includes a frame indicated generally by the reference character 12. The frame 12 includes a plurality of uprights 12a, 12b and interconnecting cross pieces 12c, 12d. A longitudinal shelf 13 extends transversely along the base of the frame and is supported by the uprights 12a, 12b. A primary support plate 15 is fastened to the uprights 12a. The support plate 15 in conjunction with the other structural members support a product receiving hopper 14, a bag feeding mechanism indicated generally by the reference character 16 and a bag loading mechanism indicated generally by the reference character 18 which also defines a loading station for a bag. The product to be packaged is conveyed to the hopper 14 by a conveyor indicated generally by the reference character 20 in Figure 1. It should be apparent that the product to be packaged drops from the conveyor 20 into the hopper 14 and is guided into an awaiting bag positioned at the loading station.

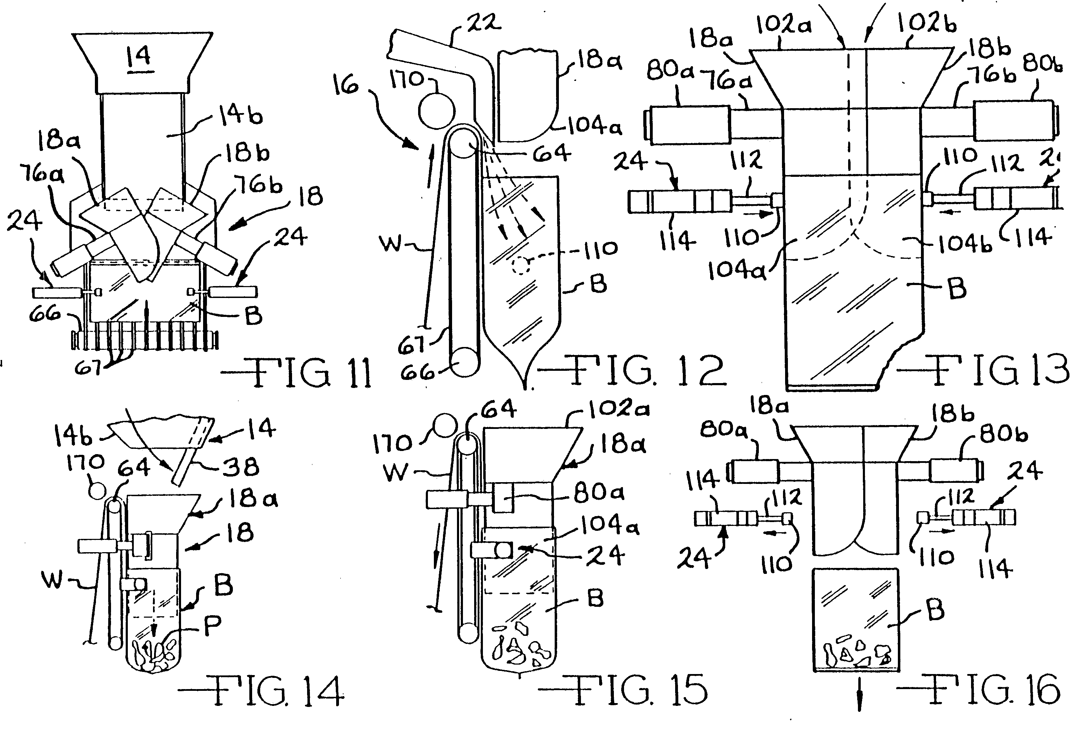

The preferred packaging process is schematically illustrated in Figures 11-16. The disclosed apparatus is operative to sequentially load a web or chain « W » of interconnected bags. A lead bag B of the chain of bags or web W is advanced towards a loading position by the conveyor mechanism 16. In the preferred construction, the loading mechanism comprises a pair of interfitting horns 18a, 18b, which together define a funnel-like structure when in the loading position.

As the bag B is being advanced, the loading mechanism 18 is retracted upwardly as shown in Figure 11. As the bag B advances to the load position, a blast of air from a nozzle 22 fed by a blower 23 (shown in Figure 2) expands the bag as shown in Figure 12. The loading mechanism is then lowered so that a bottom portion of each horn 18a, 18b enters an upper region of the bag. Grippers 24 are then actuated in order to clamp the upper region of the bag B to the side of the horns 18a, 18b. Product P drops from the hopper 14 and is guided into the bag B. The loaded bag is then severed from the web W by reversing motion in the web W as shown in Figure 15 to cause separation of the bag from the remainder of the web along a line of weakness. The grippers 24 are then retracted to release the loaded bag from the horns 18a, 18b.

Returning to Figure 1, the bagging apparatus includes a control module 30 and drive module 31 both covered by respective housings 30a, 31a. The module 30 contains electronic control circuitry for the various machine functions whereas the module 31 houses the drive system for the web and bag conveyor mechanism 16.

Referring also to Figures 2 and 3, the hopper 14 is mounted to the top of the bagger 10 and supported by frame members 32 attached to the support plate 15. An upper portion 14a of the hopper 14 defines a tapered mouth for receiving the product to be packaged. A lower region 14b defines a funnel-like shell having an exit opening 33 located just above and aligned with the horns 18a, 18b when the horns are in their product loading position as seen in Figure 2.

According to a feature of the invention, the hopper 14 includes an accumulating mechanism which in the illustrated embodiment comprises a pivotally mounted gate 38. As seen best in Figure 3, the gate 38 is movable between an open position 40b and a closed position 40a by an actuator 42 which is pivotally connected to the side of the gate 38 via a slot 44 formed in the lower section 14b of the hopper 14. The actuator 42 may be of a conventional construction and preferably of a fluid pressure operated variety. With the use of the disclosed accumulating mechanism, the conveyor 20 (shown in Figure 1) can drop product to be packaged into the hopper without interruption even during the period of time a bag B is being positioned at the loading station 18.

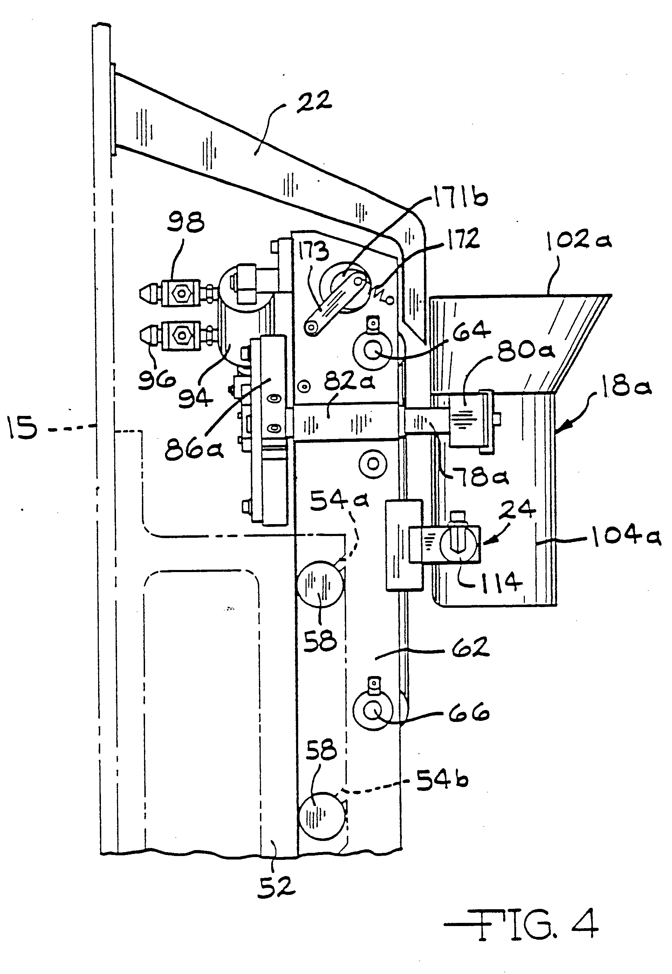

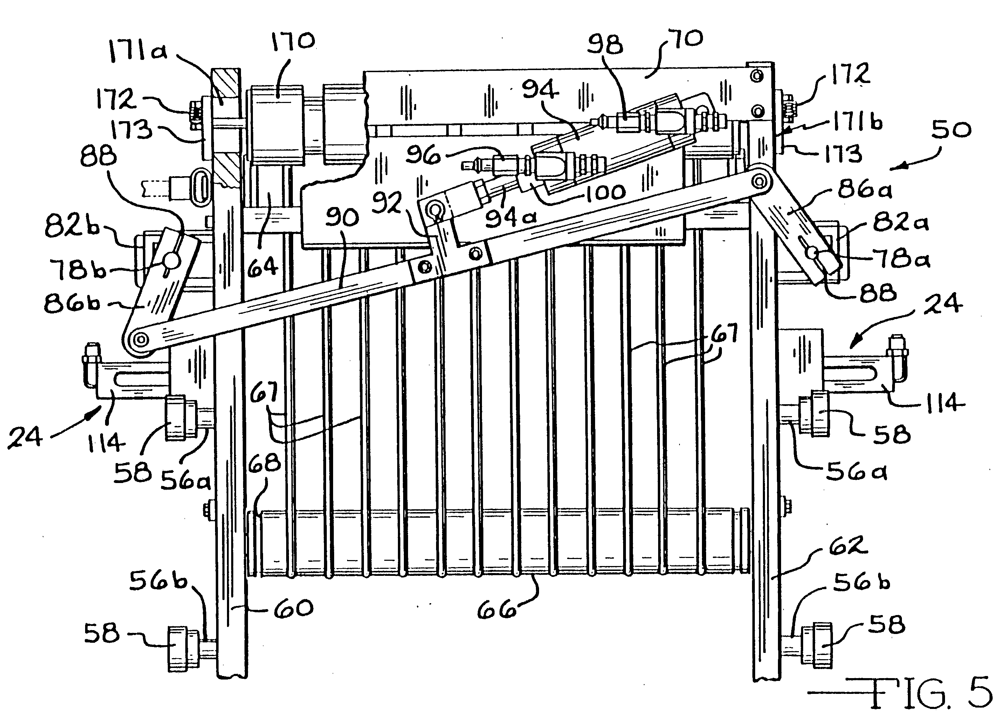

Referring to Figures 4 and 5, the bag conveyor and horn mechanism forms part of a removable assembly indicated generally by the reference character 50 in Figure 5. As seen in Figure 2, the bagging machine frame includes a pair of intermediate support plates 52 (only one is shown in Figure 2) which each include a pair of vertically spaced diagonal slots 54a, 54b. The slots are adapted to receive a pair of vertically spaced pins 56a, 56b forming part of the conveyor/horn assembly 50 as shown best in Figure 5. The pins 56a, 56b include locking knobs 58· which secure the position of the assembly 50 in the support plates 52 once the assembly is mounted.

Referring in particular to Figure 5, the rear of the conveyor is illustrated. The assembly 50 includes a pair of vertical side plates 60, 62 which journal upper and lower conveyor rollers 64, 66. A plurality of O-ring like belts 67 are reeved around the rollers, their relative spatial positions being maintained by grooves 68 formed in the rollers. A crossbar 70 is fastened to the upper ends of the side plates to maintain the side plate distance and rigidize the structure. A lower crossbar not shown serves a similar function at the lower end of the assembly.

As indicated above, the individual horns 18a, 18b, which together define a funnel-like structure when located in the load position, are pivoted between the retracted and load positions by a fluid pressure operated mechanism shown best in Figure 5. Referring also to Figures 1 and 4, the horns 18a, 18b are supported by associated arms 76a, 78b. The arms in turn are connected to shafts 78a, 78b by coupling caps 80a, 80b. The shafts 78a, 78b are journalled in associated bearing blocks 82a, 82b suitably secured to the respective side plates 60, 62.

As seen in Figure 5, rear portions of the associated shafts are engaged by lever arms 86a, 86b which each include clamping slots 88 by which the radial position of the arms on the shafts can be adjusted. The lever arms are interconnected by a coupling link 90 which includes a centrally positioned intermediate link 92. A fluid pressure operated actuator 94 pivotally mounted at one end to the crossbar 70 includes an actuating rod 94a operatively connected to the intermediate link 92 such that extension and retraction of the actuating rod transmitted to the coupling link 90 causes the lever arms 86a, 86b to concurrently rotate through predetermined arcs.

In the disclosed arrangement, when the actuating rod 94a is retracted, the horns 18a, 18b are rotated to the upper, retracted positions and when the actuator rod is extended, the horns 18a, 18b rotate downwardly to their loading positions. In the illustrated construction, the actuator is pneumatically operated and hence includes pneumatic fittings 96, 98. The actuator also includes an adjustable stop 100 for adjusting the overall stroke of the mechanism.

Returning to Figure 1, the horn 18b is sized so that a portion fits inside the horn 18a. With this construction, when the horns 18a, 18b are in the loading position, a somewhat continuous funnel-like guide is defined. Each horn also includes an upper expanded portion 102a, 102b which defines a receiving opening aligned with and slightly larger than the exit opening 33 of the hopper 14. Lower segments 104a, 104b of the respective horns 18a, 18b are somewhat cylindrical and uniform (when positioned in the loading position) so that together, the horns 18a, 18b define a cylindrical guide-like structure for guiding product into a bag.

Referring to Figures 1 and 4, a bag being loaded is clamped to the horns 18a, 18b by the grippers 24 which are secured to associated conveyor side plates 60, 62. As seen best in Figure 1, each gripper assembly includes a gripper pad 110 mounted at the end of an actuating rod 112. The gripper pad 110 and associated rod 112 are driven towards and away from the associated horn by a fluid pressure operated actuator 114. When a bag is positioned around the lower regions of the horns 18a, 18b, the gripper assemblies are actuated to drive the gripper pads 110 into abutting contact with the sides of the associated horns, thus clamping the bag to the horn assembly and isolating it from the remainder of the web as shown in Figure 13. Once the bag to be loaded is clamped to the horns, loading forces generated by the product as it drops into the bag are not transmitted to the rest of the web.

As indicated above, when the bag is loaded, it is severed from the remainder of the web by retracting the web slightly so that the web severs itself from the loaded bag along a line of weakness.

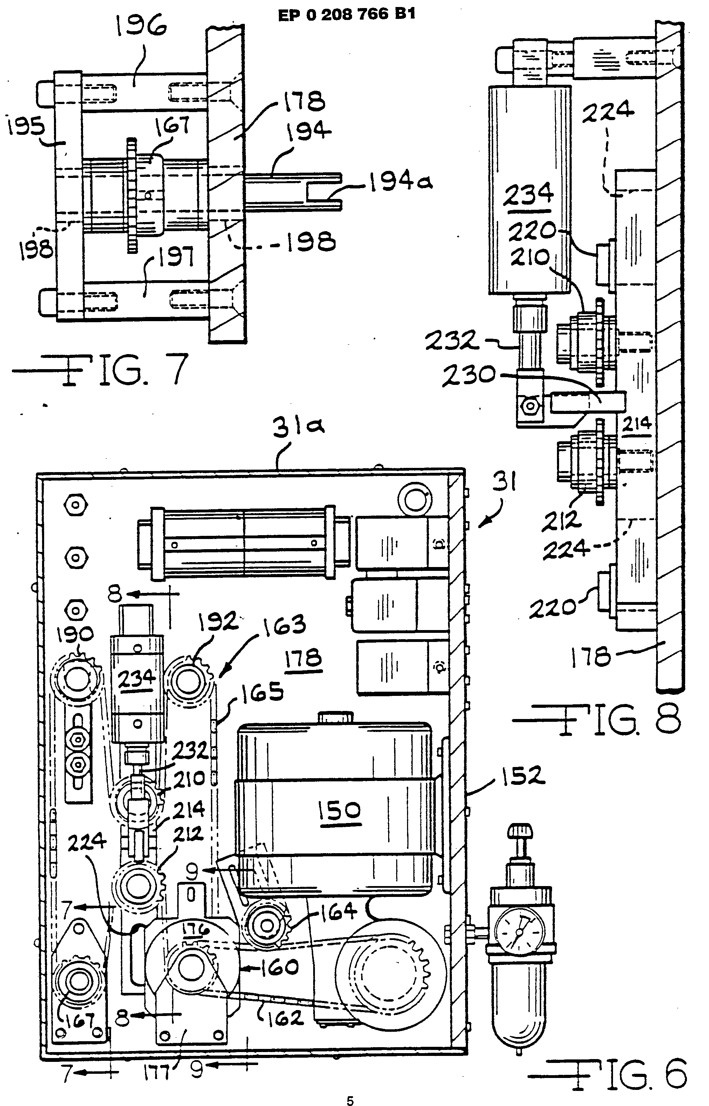

Referring to Figures 6-10, the drive system for the web/bag conveyor is illustrated. The drive system is located in the housing 31 and includes a drive motor 150 suitably mounted to a side plate 152 of the housing. In the preferred construction, the drive motor 150 is continually energized during the bagging operation even though the conveyor itself is only intermittently operated to position successive bags at the loading station. To achieve this intermittent driving arrangement, the drive motor is connected to a clutch/brake assembly 160 by a chain 162. An idler sprocket 164 is movable to adjust the tension on the chain 162.

The clutch assembly 160 controls the interconnection between the primary chain 162 and a secondary chain drive, indicated generally by the reference character 163. The secondary drive includes a chain 165 which is driven by the clutch assembly 164 when it is energized and which in turn drives a sprocket 167 connected to the upper conveyor roller 64. Referring to Figures 4 and 5, a pinch roller or feed roll 170 rotates in bearings 171a, 171b adjustably positioned in the conveyor assembly sideplates 60, 62. The feed roll 170 is biased into abutting contact with the upper conveyor roll 64 by a pair of biasing springs 172. Lever arms 173 provide a floating type engagement for the feed roll to allow the feed roll 170 to move towards and away from the upper conveyor roll 64 as the web is being fed to compensate for changes in material thickness. It should be apparent that the frictional coupling between the feed roll 170 and upper conveyor roll 64 and between the O-ring belts 67 and upper conveyor roller 64 causes the entire conveyor system including the O-ring belts 67 and lower conveyor roll 66 to rotate whenever the upper conveyor roller 64 rotates.

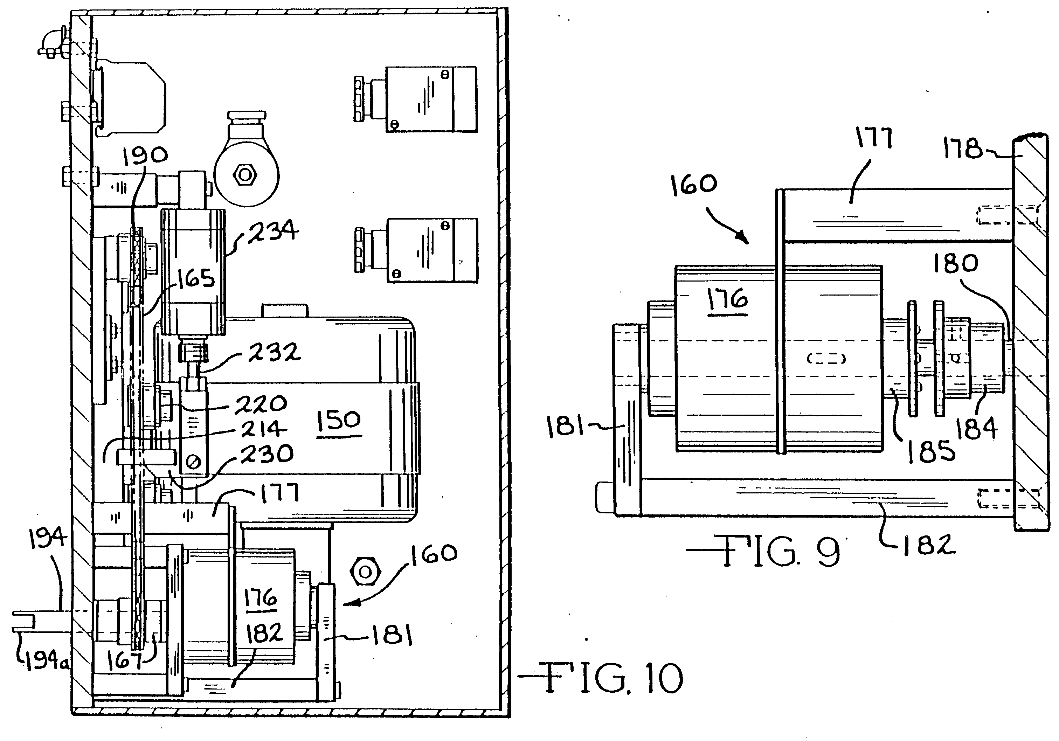

As seen in Figures 6 and 9, the clutch/brake assembly 160 is conventional and includes an electromagnetic clutch/brake unit 176 mounted to a backplate 178 of the drive housing 31 by an L-shaped bracket 177. The assembly includes a shaft 180, journalled at one end in the backplate 178 and journalled at its other end in a support piece 181 spaced from the backwall 178 by a spacing plate 182. The assembly also includes a pair of chain sprockets 184, 185. The outer sprocket 184 is fixed to the shaft 180 and is coupled to the primary chain 162 and in normal operation is continuously driven by the drive motor 150. The inner sprocket 185 forms part of the secondary drive. Its rotation is controlled by the electromagnetic unit 176. When the clutch function of the unit 176 is actuated, the sprocket 185 is frictionally coupled to the shaft 180 and hence rotates in unison with the outer sprocket 184. With the clutch actuated, the rotation in the drive motor 150 is transferred to the upper conveyor roller 64 via the sprocket 167. In the preferred construction, the direction of rotation is such that the web is driven in the forward direction whenever the clutch is actuated.

When the unit 176 is deenergized, the shaft 180 freely rotates relative to the inner sprocket 185. As indicated above, the unit 176 also includes a braking function which, when actuated, frictionally locks the inner sprocket 185 to the unit 176 to prevent or inhibit relative rotation in the sprocket 185.

Returning to Figure 6, and referring also to Figure 7, additional details of the secondary drive system 163 are illustrated. The second chain 165 is reeved-around a pair of spaced, idler sprockets 190, 192. The sprocket 190 is adjustably positioned to adjust the overall chain tension of the chain 165. The drive sprocket 167 (which drives the upper conveyor roll 64) is fastened to a stub shaft 194 (shown in Figure 7 only). The shaft 194 is journalled between the backplate 178 and a support plate 195 spaced from the backplate 178 by a pair of transversely extending spacers 196, 197. Bearings 198 rotatably support the shaft 194 in the plates. The stub shaft 194 includes a forked end 194a which interconnects with the upper conveyor roller 64 in a known manner, so that rotation of the sprocket and shaft causes rotation of the conveyor roll.

According to the invention, the lead bag in the web (indicated by the reference character B in Figure 12) is advanced to the loading position by energizing the clutch 160 to couple the drive motor 150 to the sprocket 167 via the chains 162, 165. The clutch remains energized until the bag is advanced to the load position whereupon the clutch is deenergized.

As indicated above, the loaded bag is severed from the remainder of the web by retracting the web slightly while the engagement between the loading horns 18a, 18b and the loaded bag is maintained by the grippers 24. The tension forces generated between the bag held by the gripper assemblies 24 and the rest of the web cause a severance along a line of weakness, preferably formed by a plurality of perforations located between adjacent bags. Although various reversing drive arrangements known in the art can be employed to produce this reverse feed of the web, in the preferred embodiment, a fluid pressure operated actuator is used to produce an incremental reverse rotation in the upper conveyor roll 64.

Referring in particular to Figures 6 and 8, the secondary chain drive 163 includes a pair of sprockets 210, 212 rotatably carried on a shuttle block 214. In the illustrated arrangement, the sprockets 210, 212 are journalled on pins 216 threadedly received by the shuttle block 214. The shuttle block is 214 vertically slidable along the back plate 178 and is held to the back plate by a pair of shoulder bolts 220 which extend through vertical slots 224 formed in the shuttle block 214. It should be apparent that the mounting arrangement enables vertical reciprocating motion in the shuttle 214 while resisting transverse motion. An operating arm 230 extends transversely from the shuttle block 214 and is connected to the end of an actuating rod 232 forming part of a fluid pressure actuator 234. As seen in Figure 8, when the actuating rod 232 is retracted into the actuator, the shuttle block 214 moves upwardly, whereas when the actuator rod is extended, the shuttle block moves downwardly. As seen in Figure 6, the second drive chain 165 is reeved around the shuttle sprockets 210, 212.

In order to produce reverse movement in the web W, as indicated schematically in Figure 15, the fluid pressure operated actuator 234 is energized to extend the actuating rod 232 while the brake function is applied by the clutch/brake unit 176 to prevent movement of the secondary chain 165 in the section reeved around the clutch/brake sprocket 185 and the fixed idler sprocket 192. It should be apparent that as the shuttle block 214 moves downwardly, the segment of the chain 165 reeved around the conveyor roll sprocket 167, the adjustable idler sprocket 190 and the shuttle sprockets 210, 212 is displaced and in particular produces rotation in each of these four sprockets. The downward movement of the shuttle produces clockwise movement in the conveyor roll drive sprocket 167 which causes the web held between the upper conveyor roller 64 and the spring biased feed roller 170 to reverse feed.

Since the forward end of the web is fixed by virtue of the clamped loaded bag, the generated tension in the web causes severance along a line of weakness. In normal operation, a line of perforation is located intermediate the loaded bag and the web region held between the feed roll 170 and conveyor roll 64. Severance occurs along these perforations.

Since the actuator produces rather abrupt travel in the shuttle 214, the web retraction occurs at a rapid rate causing swift and consistent separation of the web from the loaded bag. The actuator is then immediately retracted to advance the severed end of the web forwardly. If the lead bag is to be loaded, the clutch/brake unit is energized to clutch the primary drive to the secondary drive 163 to produce forward rotation of the upper conveyor roll 64.

With the preferred construction, separation of the loaded bag from the remainder of the web can be done reliably and quickly. Moreover, by using a quick acting actuator mechanism, the feed rate of the machine is substantially improved over a system that would require reverse rotation of either the drive motor or the secondary drive chain via a clutch/brake system. As indicated above, with the preferred method, the drive motor (50) rotates continuously throughout the bagger operation and is merely clutched or declutched from the conveyor drive system in order to control advancement of the web.

The disclosed bagging apparatus and method is expecially suited for packaging poultry parts.

The product conveyor (20) (illustrated in Figure 1 only) operates continuously and transports cut poultry carcasses to the bagging machine (10) for packaging into bags. In normal operation, individual bins (not shown) of the conveyor (20) each include a single poultry carcass divided into eight or nine parts. The divided carcass is deposited on the conveyor (20) at an unloading station of the cutting machine. It has been found desirable to package multiple carcasses in a single bag. The hopper/gate mechanism (38) shown in Figures 1-3 enables multiple products to be packaged in a single bag. As indicated previously, a fluid pressure operated actuator (42) controls the position of the gate (38). Referring also to Figures 14-16, the gate (38) is moved to the open position (40a) once the lead bag (B) has been clamped to the sides of the horns 18a, 18b by the gripper assemblies 24. As soon as the loading of the bag has been completed, the gate actuator 42 retracts in order to close off the exit opening 33 of the hopper 14. Any product discharged by the conveyor 20 is then retained in the hopper. During the period of time that the gate 38 is closed, the loaded bag is severed from the web and dropped from the horns 18a, 18b. The web is then advanced to position the next bag at the load position. Once the gripper assemblies 24 are actuated to clamp the bag to the horns, the gate actuator is then extended to open the gate 39 to allow the product to drop into the waiting bag. It should be apparent that depending on the speed of the conveyor 20, one or more products can be discharged into the hopper 14 during the period of time the gate 38 is closed and hence the number of products loaded into a single bag can be adjusted by controlling the period of time the gate 38 is closed and/or the speed of the conveyor 20.

Although the invention has been described with a certain degree of particularity, it should be understood that those skilled in the art can make various changes to it without departing from the spirit or scope of the invention as hereinafter claimed.

## Claims

1. A packaging machine for loading and separating a bag-like container from a chain or web of containers, comprising

a) a structure (64, 66, 67) establishing a path of travel for a web (W) of interconnected bag-like containers,

b) means (170) for advancing said web (W) along said path to successively position each of said containers at a loading station,

c) means (22) for expanding a container (B) positioned at said loading station, and

d) a container loading mechanism comprising funnel means (18) cooperable with the expanded container (B) and operative to funnel a product into the container, characterised by

e) said funnel means being movable into said expanded container,

f) gripper means (24) operative to immobilize said container (B) at said loading station to inhibit relative movement between said container (B) and said loading mechanism (18), and

g) container separating means for severing said container (B) from said web (W), comprising means (214, 230, 232, 234) for reversing movement in said web (W) while said container is immobilized, said separating means being operative to retract the web to cause separation along a line of weakness connecting said loaded container to the web.

2. A machine according to claim 1 wherein said means (22) for expanding said container comprises means for delivering a burst of relatively high velocity air.

3. A machine according to claim 1 or 2 wherein the gripper means (24) is operable to clamp a face of said container (B) to said container loading mechanism (18).

4. A machine according to claim 1, 2 or 3 wherein the container loading mechanism (18) comprises a pair of pivotally mounted horns (18a, 18b) which together define a funnel-shaped structure when pivoted to a loading position.

5. A machine according to any one of the preceding claims wherein said means for reversing movement in said web comprises a fluid pressure operated actuator (234) operatively connected to a web drive roller (170) such that upon actuation said actuator (234) reverse rotates said drive roller (170).

6. A machine according to any one of the preceding claims further including a feed hopper (14) including a closure gate (38) for holding articles to be packaged until a container has been positioned and opened at said loading station.

7. A method for loading and separating a bag-like container from a chain or web of containers comprising the steps of

a) providing a web (W) of packaging material defining a plurality of containers, each container joined to the web of containers by a line of weakness ;

b) advancing said web along a path (64, 66, 67) to successively position each of said containers at a loading station ;

c) expanding the container positioned at the loading station to an open condition ;

d) positioning a funnel means (18) of a container loading mechanism and the container (B) at the loading position with the loading mechanism extending into the opening defined by said container, and

e) loading said open container, characterised by the steps of

f) moving the funnel means into the opening defined by said container prior to loading ;

g) immobilizing the container at the loading station by the operation of gripper means (24) to inhibit relative movement between the container and said loading mechanism during loading ;

h) retracting said web a predetermined distance while the gripper means is operative to cause separation of the web from the loaded container along the line of weakness connecting the container to the web, and

i) releasing the loaded and separated container for discharge from the loading station.

8. The method of claim 7 wherein said step of opening said container is achieved by a burst of high velocity air.

9. The method of claim 7 further comprising the step of holding articles to be packaged in an accumulating means until a container has been positioned and opened at said loading station.

10. A machine according to any one of claims 1 to 6 in which the means for advancing the web comprises

a) a pinch roller assembly defined by a conveyor roller (64) and an associated feed roller (170) biased into contact with said conveyor roller such that said rollers drivingly engage a web (W) positioned between said rollers, and

b) a roller drive mechanism comprising

i) a drive source (150) and a clutch means (160) for selectively coupling said drive source (150) with said conveyor roller (64) to drive said web (W) in an advancing direction and wherein the means for reversing movement of the web comprises

ii) reversing means including means for incrementally reverse rotating said conveyor roller to produce a retraction in a portion of said web, including a fluid pressure operated actuator (234) and means (214, 230, 232) for transferring rectilinear motion in said actuator (234) to rotational movement in said conveyor roller (64).

11. A machine according to claim 10 wherein said drive mechanism comprises first and second drive chains (162, 165) said first chain (162) connecting said drive source (150) to said clutch means (160) and said second chain (165) connecting an output of said clutch means (160) with said conveyor roller (64) such that actuation of said clutch interconnects said first and second drive chains (162, 165).

12. A machine according to claim 11 wherein said second drive chain (165) is operatively connected to a reciprocal shuttle (214) such that upon actuation of said actuator (234) said shuttle (214) is displaced and produces incremental translation in said second drive chain (165) to produce reverse rotation in said conveyor roller (64).

**Patentansprüche**

1. Verpackungsmaschine zum Füllen und Abtrennen eines beutelartigen Behälters von einer Kette oder einer Bahn von Behältern, welche Maschine

a) eine eine Laufbahn für eine Bahn (W) von miteinander verbundenen beutelartigen Behältern bildende Struktur (64, 66, 67)

b) Mittel (170) zum Vorwärtsbewegen der genannten Bahn (W) längs dieser Laufbahn um nacheinander jeden der Behälter an einer Füllstation zu positionieren,

c) Mittel (22) zum Aufweiten eines an der Füllstation befindlichen Behälters (B), und

d) eine mit Trichtermitteln versehene Behälterfüllvorrichtung (18), welche mit dem aufgeweiteten Behälter (B) zusammenarbeitet und mit der ein Produkt in den Behälter einfüllbar ist, aufweist, dadurch gekennzeichnet, daß

e) die Trichtermittel in den aufgeweiteten Behälter einführbar sind, daß

f) Greifermittel (24) vorgesehen sind, mit denen der Behälter (B) an der Füllstation stillsetzbar ist, um eine Relativbewegung zwischen dem Behälter (B) und der Füllvorrichtung (18) zu verhindern, und daß

g) Behältertrennmittel zum Abtrennen der Behälter (B) von der Bahn (W) vorgesehen sind, welche Mittel (214, 230, 232, 234) zum Umkehren der Bewegung der Bahn (W), während der Behälter zum Stillstand gebracht ist, aufweisen, mit welchen Trennmitteln die Bahn zurückziehbar ist, um eine Trennung längs einer Schwächungslinie herbeizuführen, welche den befüllten Behälter mit der Bahn verbindet.

2. Maschine nach Anspruch 1, dadurch gekennzeichnet, daß die Mittel (22) zum Aufweiten der Behälter Mittel zum Abgeben eines Stoßes von Luft mit verhältnismäßig großer Geschwindigkeit aufweisen.

3. Maschine nach Anspruch 1 oder 2, dadurch gekennzeichnet, daß mit den Greifermitteln (24) eine Seite des Behälters (B) an der Behälterfüllvorrichtung (18) festklemmbar ist.

4. Maschine nach Anspruch 1, 2 oder 3, dadurch gekennzeichnet, daß die Behälterfüllvorrichtung (18) ein Paar schwenkbar angebrachter Hörner (18a, 18b) aufweist, welche zusammen eine trichterförmige Anordnung bilden, wenn sie in eine Füllstellung geschwenkt sind.

5. Maschine nach einem der vorhergehenden Ansprüche, dadurch gekennzeichnet, daß die Mittel zum Umkehren der Bewegung der Bahn eine mit Fluiddruck betriebene Betätigungsvorrichtung (234) aufweisen, die mit einer Bahnantriebswalze (170) wirkverbunden ist, so daß nach Auslösen die Betätigungsvorrichtung (234) die Antriebswalze (170) in umgekehrter Richtung in Drehung versetzt.

6. Maschine nach einem der vorhergehenden Ansprüche, dadurch gekennzeichnet, daß sie weiter einen Speisetrichter (14) aufweist, der ein Verschlußtor (38) besitzt, um zu verpackende Gegenstände zurückzuhalten, bis ein Behälter an der betreffenden Füllstation positioniert und geöffnet worden ist.

7. Verfahren zum Füllen eines beutelartigen Behälters und zum Trennen desselben von einer Kette oder einer Bahn von Behältern, welches Verfahren die Schritte :

a) Vorsehen einer Bahn (W) aus Verpackungsmaterial, welche eine Vielzahl von Behältern bildet, wobei jeder Behälter mit der Behälterbahn über eine Schwächungslinie verbunden ist,

b) Vorwärtsbewegen der Bahn längs einer Laufbahn (64, 66, 67) um nacheinander jeden der Behälter an einer Füllstation zu positionieren,

c) Aufweiten des an der Füllstation positionierten Behälters zu einem offenen Zustand,

d) Positionieren eines Trichtermittels (18) einer Behälterfüllvorrichtung und des Behälters (B) an der Füllstellung, wobei die Füllvorrichtung in die durch den Behälter begrenzte Öffnung ragt, und

e) Füllen des offenen Behälters, aufweist, gekennzeichnet durch die Schritte :

f) Einführen der Trichtermittel in die durch den Behälter begrenzte Öffnung vor dem Füllen,

g) Stillsetzen des Behälters an der Füllstation durch Greifmittel (24), um eine Relativbewegung zwischen dem Behälter und der Füllvorrichtung während des Befüllens zu verhindern,

h) Zurückziehen der Bahn um eine vorbestimmte Strecke während die Greifmittel in Wirkung sind, um ein Trennen der Bahn vom befüllten Behälter längs der Schwächungslinie, die den Behälter mit der Bahn verbindet, herbeizuführen, und

i) Freigeben des befüllten und abgetrennten Behälters zur Ausgabe von der Füllstation.

8. Verfahren nach Anspruch 7, dadurch gekennzeichnet, daß der Schritt des Öffnens des Behälters durch einen Stoß von Luft mit hoher Geschwindigkeit ausgeführt wird.

9. Verfahren nach Anspruch 7, welches weiter den Schritt aufweist, daß die zu verpackenden Gegenstände in einer Sammeleinrichtung (14) gehalten werden, bis ein Behälter an der Füllstation positioniert und geöffnet worden ist.

10. Maschine nach einem der Ansprüche 1 bis 6, dadurch gekennzeichnet, daß die Mittel zum Vorwärtsbewegen der Bahn

a) eine Klemmwalzenanordnung, welche durch eine Transportwalze (64) und eine zugeordnete Andruckwalze (170) gebildet ist, welche so zum Anliegen an der Transportwalze vorbelastet ist, daß die genannten Walzen eine Bahn (W), welche zwischen diesen Walzen angeordnet ist, mitnehmen, und

b) eine Walzenantriebsvorrichtung aufweisen, welche

i) eine Antriebsquelle (150) und eine Kupplungseinrichtung (160) zum wahlweisen Kuppeln der Antriebsquelle (150) mit der Transportwalze (64), um die Bahn (W) in vorlaufender Richtung anzutreiben, besitzt, und daß die Mittel zum Umlenken der Bewegung der Bahn

ii) Umkehrmittel aufweisen, die Mittel zum schrittweisen Drehen der Transportwalze in Gegenrichtung, um ein Zurückschieben in einem Teil der Bahn herbeizuführen, umfassen, welche mit einer mit Fluiddruck betriebenen Betätigungsvorrichtung (234) und mit Mitteln (214, 230, 232) zum Umformen einer geradlinigen Bewegung der Betätigungsvorrichtung (234) in eine Drehbewegung der Transportwalze (64) versehen sind.

11. Maschine nach Anspruch 10, dadurch gekennzeichnet, daß die Antriebsvorrichtung erste und zweite Antriebsketten (162, 165) aufweist, wobei die erste Kette (162) die Antriebsquelle (150) mit der Kupplungseinrichtung (160) und die zweite Antriebskette (165) einen Ausgang der Kupplungseinrichtung (160) mit der Transportwalze (64) verbindet, so daß ein Betätigen der Kupplung die erste und die zweite Antriebskette (162, 165) miteinander verbindet.

12. Maschine nach Anspruch 11, dadurch gekennzeichnet, daß die zweite Antriebskette (165) mit einem hin- und hergehenden Schieber (214) wirkverbunden ist, so daß beim Auslösen der Betätigungsvorrichtung (234) der Schieber (214) versetzt wird und eine schrittweise Verschiebung der zweiten Antriebskette (165) erzeugt, um eine Drehung der Transportwalze (64) in Gegenrichtung herbeizuführen.


**Revendications**

1. Une machine d'emballage pour charger et séparer un conteneur du type sac à partir d'une chaîne ou d'un tissu de conteneurs, comprenant :
a) une structure (64, 66, 67) établissant un chemin de parcours pour un tissu (W) de conteneurs du type sac reliés l'un à l'autre
b) des moyens (170) pour faire avancer ledit tissu (W) le long dudit chemin pour placer successivement chacun desdits conteneurs à une station de chargement et
c) des moyens (22) pour dilater un conteneur (B) placé à ladite station de chargement et
d) un mécanisme de chargement du conteneur comprenant des moyens d'entonnoir (18) capables de coopérer avec le conteneur (B) dilaté et actifs pour canaliser un produit vers le conteneur,
e) lesdits moyens d'entonnoir étant déplaçables dans ledit conteneur dilaté
f) des moyens de mâchoires (24) actifs pour immobiliser ledit conteneur (B) à ladite station de chargement pour inhiber le mouvement relatif entre ledit conteneur et ledit mécanisme de chargement (18), et
g) des moyens de séparation du conteneur pour détacher ledit conteneur (B) dudit tissu (W), comprenant des moyens (214, 230, 232, 234) pour inverser le mouvement dudit tissu (W) tandis que ledit conteneur est immobilisé, lesdits moyens de séparation étant actifs pour tirer en arrière le tissu pour entraîner la séparation le long d'une ligne de fragilité reliant au tissu ledit conteneur chargé.

2. Une machine selon la revendication 1 dans laquelle lesdits moyens (22) pour dilater ledit conteneur comprend des moyens pour délivrer un jet d'air de vitesse relativement élevée.

3. Une machine selon la revendication 1 ou 2 dans laquelle les moyens de mâchoires (24) sont capables d'agir pour bloquer une face dudit conteneur (B) audit mécanisme (18) de chargement du conteneur.

4. Une machine selon la revendication 1, 2 ou 3 dans laquelle le mécanisme de chargement du conteneur (18) comprend une paire de cornets montés de façon pivotante (18a, 18b) qui, ensemble, définissent une structure en forme d'entonnoir quand ils sont tournés dans une position de chargement.

5. Une machine selon n'importe laquelle des revendications précédentes dans laquelle lesdits moyens pour inverser le mouvement dudit tissu comprend un actionneur (234) fonctionnant par pression de fluide connecté en fonctionnement à un galet d'entraînement du tissu (170) de sorte que sur commande ledit actionneur (234) tourne en sens inverse ledit galet d'entraînement (170).

6. Une machine selon n'importe laquelle des revendications précédentes incluant de plus une trémie d'alimentation (14) incluant une porte de fermeture (38) pour retenir les objets devant être emballés jusqu'à ce qu'un conteneur ait été placé et ouvert à ladite station de chargement.

7. Une méthode pour charger et séparer un conteneur de type sac à partir d'une chaîne ou d'un tissu de conteneurs comprenant les étapes de
a) fourniture d'un tissu (W) de matériau d'emballage définissant une pluralité de conteneurs, chaque conteneur étant relié au tissu de conteneurs par une ligne de fragilité ;
b) avance dudit tissu le long d'un chemin (64, 66, 67) pour placer successivement chacun desdits conteneurs une station de chargement
c) dilatation du conteneur placé à la station de chargement en condition d'ouverture ;
d) mise en place des moyens d'entonnoirs d'un mécanisme de chargement d'un conteneur et le conteneur (B) à la position de chargement avec le mécanisme de chargement se prolongeant dans l'ouverture définie par ledit conteneur, et
e) chargement dudit contenu caractérisé par les étapes de
f) déplacement des moyens d'entonnoir dans l'ouverture définie par ledit conteneur avant le chargement ;
g) immobilisation du conteneur à la station de chargement par l'action des moyens de mâchoires (24) pour inhiber le mouvement relatif entre le conteneur et ledit mécanisme de chargement pendant le chargement ;
h) retrait du tissu d'une distance prédéterminée tandis que les moyens de mâchoires sont en action pour entraîner la séparation du tissu du conteneur chargé le long de la ligne de fragilité reliant le conteneur au tissu, et
i) relâchement du conteneur chargé et séparé pour libération de la station de chargement.

8. La méthode de la revendication 7 dans laquelle ladite étape d'ouverture dudit conteneur est réalisée par un jet d'air de vitesse élevée.

9. La méthode de la revendication 7 comprenant en plus l'étape de retenue des objets devant être emballés par des moyens d'accumulation (14) jusqu'à ce qu'un conteneur ait été placé et ouvert à ladite station de chargement.

10. Une machine selon n'importe laquelle des revendications 1 à 6 dans laquelle les moyens pour faire avancer le tissu comprennent

a) un assemblage, de galet presseur défini par un galet transporteur (64) et un galet d'entraînement associé (170) prédisposé en contact avec ledit galet transporteur de telle sorte que lesdits galets engagent par entraînement un tissu (W) placé entre lesdits galets, et

b) un mécanisme d'entraînement par galet comprenant

i) une source d'entraînement (150) et des moyens d'accouplement (160) pour coupler sélectivement ladite source d'entraînement (150) audit galet transporteur (64) pour entraîner ledit tissu (W) dans une direction d'avancement et dans lequel les moyens pour inverser le mouvement du tissu comprend

ii) des moyens d'inversion incluant des moyens de rotation inverse par incrément dudit galet transporteur pour produire un retrait dans une partie dudit tissu, incluant un actionneur fonctionnant par pression fluide (234) et des moyens (214, 230, 232) pour transférer le mouvement rectiligne dans ledit actionneur (234) en un mouvement de rotation dans ledit galet transporteur (64).

11. Une machine selon la revendication 10 dans laquelle ledit mécanisme d'entraînement comprend une première et une seconde chaîne d'entraînement (162, 165), ladite première chaîne (162) reliant ladite sources d'entraînement (150) auxdits moyens d'accouplement (160) et ladite seconde chaîne (165) reliant une sortie desdits moyens d'accouplement (160) audit galet transporteur (64) de telle sorte que la mise en action dudit accouplement connecte l'une à l'autre lesdites première et seconde chaînes d'entraînement (162, 165).

12. Une machine selon la revendication 11 dans laquelle ladite seconde chaîne d'entraînement (165) est fonctionnellement reliée à un dispositif de va-et-vient (214) de telle sorte que par mise en action dudit actionneur (234) ledit dispositif de va-et-vient (214) est déplacé et produit une translation incrémentielle dans ladite seconde chaîne (165) pour produire une rotation inverse dans ledit galet transporteur (64).

FIG. 1

FIG.3

FIG. 2

EP 0 208 766 B1

FIG. 4

FIG. 5

EP 0 208 766 B1

FIG. 7

FIG. 8

FIG. 6

FIG. 9

FIG. 10

EP 0 208 766 B1

FIG 11

FIG. 12

FIG 13

FIG. 14

FIG. 15

FIG. 16

EP 0 208 766 B1